# FASCICULE DE BREVET EUROPEEN

(11) **EP 2 190 340 B1**
(45) Date de publication et mention de la délivrance du brevet: **05.10.2016**
(21) Numéro de dépôt: 08787336.0
(22) Date de dépôt: 20.08.2008
(51) Int. Cl.: A61B 1/00

(54) **SYSTÈME D'IMAGERIE POUR L'OBSERVATION TRIDIMENSIONNELLE D'UN CHAMP OPÉRATOIRE**
BILDDARSTELLUNGSSYSTEM ZUR DREIDIMENSIONALEN BEOBACHTUNG EINES OPERATIONSFELDS
IMAGING SYSTEM FOR THE THREE DIMENSIONAL OBSERVATION OF AN OPERATION FIELD

(30) Priorité: 24.08.2007 FR 0757160
(43) Date de publication de la demande: 02.06.2010
(73) Titulaire: Université Grenoble Alpes, 38400 Saint-Martin-d'Hères (FR)
(72) Inventeur: CINQUIN, Philippe, F-38330 St Nazaire Les Eymes (FR); VOROS, Sandrine, F-92310 Sevres (FR); BOSCHET, Christophe, F-83130 La Garde (FR); FOUARD, Céline, F-38100 Grenoble (FR); MOREAU-GAUDRY, Alexandre, F-38000 Grenoble (FR)
(74) Mandataire: Regimbeau
(86) Numéro de dépôt international: PCT/EP2008/060871
(87) Numéro de publication internationale: WO 2009/027278

(56) Documents cités:
- EP-A- 1 690 497
- WO-A-2007/078003
- US-A- 5 647 838
- US-A- 5 749 362
- US-A1- 2002 007 110
- US-A1- 2003 176 794
- US-A1- 2004 002 627
- US-A1- 2005 234 294

## Description

### DOMAINE DE L'INVENTION

La présente invention concerne le domaine de l'imagerie médicale, et plus particulièrement de la visualisation d'organes par endoscopie lors d'opération chirurgicales.

### ETAT DE LA TECHNIQUE

L'endoscopie chirurgicale est une technique d'exploration médicale minimalement invasive qui permet, tout en assurant une réparation identique aux techniques conventionnelles, de diminuer la taille des incisions et de limiter les zones de dissection. On l'appelle chirurgie laparoscopique dans le cas de la chirurgie minimale invasive à travers la cavité abdominale. Cette technique de chirurgie réduit considérablement le traumatisme opératoire, le risque de complication, la douleur et le temps de récupération des patients, et limite le préjudice esthétique.

La chirurgie endoscopique reste néanmoins difficile à réaliser et requiert des procédures d'apprentissage spécifiques. Pour le chirurgien, la vision directe du site opératoire est remplacée par une image 2D sur un écran via un endoscope avec les limitations que cela induit : éclairement imparfait, zones d'ombre, perception du relief difficile, champ de vision limité, occlusions possibles avec des instruments cachés par des organes. Ce système visuel peut présenter des déformations géométriques et est limité en résolution, en contraste et en couleur.

Il a par conséquent été développé différents systèmes de visualisation tridimensionnelle (3D) afin de recréer la perception de la profondeur, de manière à améliorer la perception des organes dans le champ opératoire.

La solution la plus simple et la plus économique pour faire apparaître la profondeur dans l'image endoscopique est de créer des ombres. Le fait d'ajouter une lumière indirecte en plus de la source de lumière située à l'extrémité de l'endoscope permet de projeter des ombres sur les structures visualisées, recréant ainsi une nouvelle sensation de profondeur. Un tel système de visualisation est toutefois peu performant et ne donne pas des qualités d'image satisfaisantes pour le praticien.

Une autre solution consiste à utiliser un stéréo-endoscope couplé à des lunettes polarisantes. Un stéréo-endoscope consiste en un endoscope unique dans lequel sont assemblés deux systèmes optiques distincts et deux caméras, permettant de capturer des images légèrement différentes d'une même région, et imitant ainsi la vision humaine. Les images issues du stéréo-endoscope sont projetées alternativement sur un écran unique à une fréquence de 100 Hz. L'écran est polarisé verticalement et ensuite horizontalement par un filtre polarisant actif. Les lunettes polarisantes ont un verre polarisé verticalement et un autre polarisé horizontalement de telle manière que chaque oeil ne reçoive qu'une seule de ces deux images, permettant ainsi au chirurgien d'avoir une visualisation 3D de la région observée. Néanmoins ce type de système comporte un certain nombre d'inconvénients, liés d'une part au stéréo-endoscope qui ne permet des observations que sur de très petites distances, et d'autre part à l'utilisation des lunettes qui fatiguent les yeux, de sorte que cette solution est difficilement utilisable pour des opérations pouvant durer plusieurs heures. En outre, la qualité d'image reste inférieure aux systèmes de visualisation bidimensionnelle (2D) existants.

Un système endoscopique d'imagerie 3D utilisant de la lumière structurée a également été développé. Il est composé de deux canaux, l'un permettant d'obtenir une image, l'autre de projeter une lumière structurée. Structurer de la lumière consiste à projeter une lumière, blanche en général, à travers des réseaux de lignes afin de créer une trame sur l'objet à acquérir. L'objet est visualisé par une caméra, telle qu'une caméra CCD (caméra à couplage de charges), qui observe l'objet au travers d'une trame légèrement décalée afin de créer un effet de Moiré. La répartition et l'intensité de cet effet mettent en valeur la volumétrie de l'objet et permettent l'acquisition de la troisième dimension. Néanmoins un tel système est difficile à implémenter, et est surtout coûteux.

Outre les inconvénients déjà cités pour chacune des solutions de visualisation 3D existantes, ces systèmes n'offrent pas la qualité de visualisation espérée, une gêne visuelle étant toujours présente. La chirurgie endoscopique imposant déjà des contraintes importantes, les systèmes de visualisation 2D sont plus fiables, et restent en conséquence la solution privilégiée par les chirurgiens, malgré les inconvénients liés à leur utilisation.

Il a par ailleurs été proposé des systèmes d'imagerie prévus pour construire une image tridimensionnelle directement visualisable par le praticien à partir d'informations issues de deux caméras endoscopiques, généralement manipulées également par le praticien. Les documents US 5,749,362, US 2005/234294 et US 5,647,838 donnent des exemples de réalisation de tels systèmes d'imagerie.

Dans tous les cas, de tels systèmes de visualisation pour chirurgie endoscopique sont relativement complexes à mettre en oeuvre puisque le praticien doit, en même temps qu'il effectue les opérations chirurgicales, déplacer l'endoscope en fonction du champ de visualisation désiré, ou commander un assistant pour qu'il déplace l'endoscope selon le champ de visualisation désiré.

Un but de la présente invention est donc de proposer un système endoscopique d'imagerie permettant une visualisation tridimensionnelle d'un champ opératoire, qui est simple et qui permet de résoudre au moins l'un des inconvénients précités.

### EXPOSE DE L'INVENTION

A cette fin, on propose un système endoscopique d'imagerie pour l'observation d'un champ opératoire dans un volume situé à l'intérieur du corps d'un animal, tel que défini par la revendication 1.

### DESCRIPTION DES FIGURES

D'autres caractéristiques et avantages ressortiront encore de la description qui suit, laquelle est purement illustrative et non limitative et doit être lue en regard des figures annexées parmi lesquelles :
- La figure 1 est une représentation schématique du système endoscopique d'imagerie selon un mode de réalisation de l'invention ;
- La figure 2 est une représentation schématique du système endoscopique d'imagerie selon un autre mode de réalisation de l'invention ;
- La figure 3 est un diagramme illustrant le fonctionnement du système endoscopique d'imagerie ;
- La figure 4 est une représentation schématique du système endoscopique d'imagerie selon l'invention avec un dispositif de projection de détermination relative du positionnement des caméras.

### DESCRIPTION DETAILLEE DE L'INVENTION

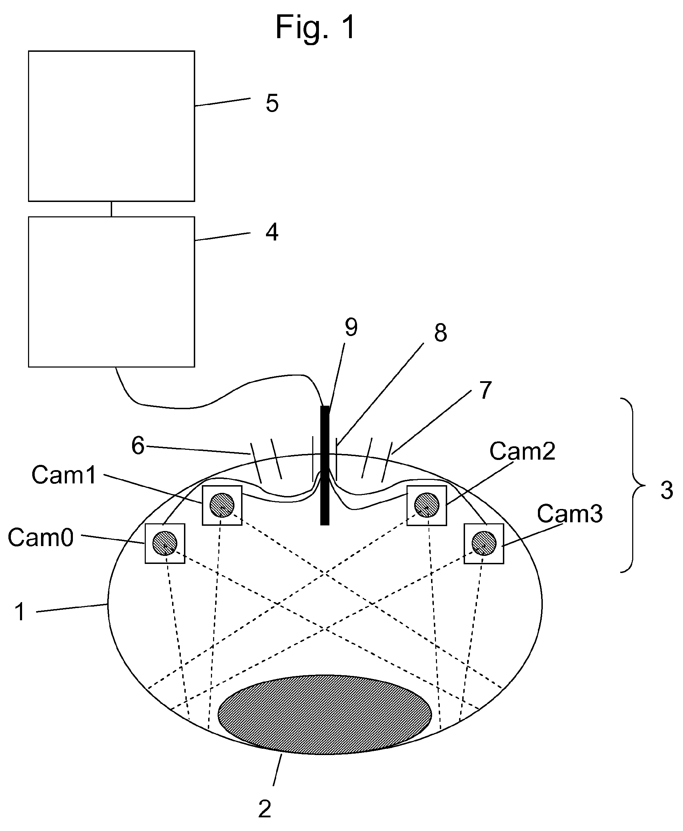

La figure 1 est une représentation schématique du système endoscopique d'imagerie proposé.

Ce système d'imagerie comprend des moyens de capture d'images 3 qui sont adaptés pour être insérés par voie endoscopique dans un volume 1 située à l'intérieur du corps d'un animal, tel qu'un humain, qui a besoin de subir une intervention chirurgicale au niveau d'un champ opératoire 2. Ce volume 1, ou région volumique, forme une cavité 1, cette cavité étant naturelle ou artificielle créée dans ce cas par injection d'air dans le volume.

Les moyens de capture 3 sont reliés à des moyens de traitement d'information 4, qui se matérialisent typiquement à une unité centrale dotée de moyens de calcul tels qu'un microprocesseur, de manière à pouvoir traiter l'information issue des moyens de capture 3.

Les moyens de traitement 4 sont en outre couplés à des moyens d'affichage 5 qui permettent une visualisation des informations issues des moyens de traitement 4, ces informations étant typiquement des informations tridimensionnelles sur le champ opératoire 2 de sorte que le praticien puisse visualiser le champ opératoire 2 en même temps qu'il effectue son intervention chirurgicale.

Comme cela est illustré schématiquement à la figure 1, il est pratiqué dans la cavité 1 une ou plusieurs incisions, qui serviront à l'insertion des outils chirurgicaux, ainsi qu'à l'insertion des moyens de capture d'images. Ces incisions sont réalisées à l'aide de trocarts (6;7;8) qui sont insérés à travers la paroi formant la cavité 1, et qui serviront de moyens de guidage pour les éléments à introduire à l'intérieur de la cavité 1.

Typiquement on prévoit deux trocarts (6;7) qui seront dédiés au passage des outils (non représentés) du praticien lors de l'opération chirurgicale.

On prévoit en outre au moins un trocart 8 supplémentaire pour l'introduction des moyens de capture 3 dans la cavité 1, de manière à pourvoir visualiser la zone intéressante du champ opératoire 2. Il est à noter toutefois qu'on pourrait envisager d'introduire les moyens de capture d'images par les deux trocarts (6;7) prévus pour les outils sans avoir en conséquence à avoir un trocart 8 dédié.

Les moyens de capture 3 du système endoscopique d'imagerie proposé comprennent une pluralité de dispositifs de capture d'images destinés à être introduits et déployés à l'intérieur de la cavité 1 du patient. On utilisera préférentiellement comme dispositifs de capture d'images des caméras. Même si la suite de la description se réfère à ce mode de réalisation particulier, il pourra être utilisé d'autres dispositifs, comme des dispositifs d'imageries par ultrasons de type sondes échographiques.

Le principe est donc d'agencer un certain nombre de caméras à l'intérieur de la cavité 1 de manière à pouvoir obtenir des informations tridimensionnelles du champ opératoire 2 selon plusieurs points de vue, sans avoir à déplacer ces caméras comme cela est le cas pour toutes les solutions invasives connues.

Un mode particulier de l'invention consiste à coupler deux par deux ces caméras, formant ainsi une pluralité de couples de caméras. Chaque couple de caméras forme un système de vision stéréoscopique, les informations collectées par ces caméras pouvant en effet être corrélées pour fournir une information 3D sur une zone particulière du champ opératoire 2. La vision stéréoscopique constitue en effet une approche privilégiée pour le rendu de la perception 3D. Elle s'appuie sur la disparité binoculaire : chaque oeil capte une vision du monde selon un point de vue différent. La mise en correspondance des disparités de ces deux images permet d'estimer la distance entre les objets observés et le point d'observation. Dans le cadre de la vision par ordinateur, on utilise deux images prises par deux caméras au lieu des images perçues par chacun des deux yeux. Un traitement adéquat de ces images permet d'obtenir une information 3D sur une zone particulière observée, voire de faire une reconstruction d'image 3D entière de la zone observée. Cette reconstruction informatique est particulièrement avantageuse puisqu'elle permet de fournir directement une représentation 3D du champ opératoire au chirurgien, sans que son cerveau ne soit sollicité pour effectuer lui-même cette représentation. Elle fournit également la base à partir de laquelle il est possible de choisir un point de vue virtuel quelconque, ainsi que les conditions d'éclairage les mieux adaptées, par des techniques de « synthèse d'images » classiques. On reviendra plus tard sur le fonctionnement du capteur stéréoscopique formé par chaque couple de caméras.

Les moyens de capture 3 comprennent donc plusieurs caméras de petites dimensions de manière à pouvoir être insérées dans la cavité 1 via un trocart ménagé à travers la paroi de la cavité 1. Le volume des caméras est typiquement que quelques millimètres-cube (mm³), préférentiellement inférieur à 5 mm³. En outre les caméras utilisées ont des optiques performantes, basées sur des technologies CMOS (acronyme de « complementary metal-oxide semiconductor » pour « semi-conducteur à oxyde de métal complémentaire ») ou CCD (acronyme de « charge-coupled device » pour « dispositif à couplage de charges »).

Les caméras sont indépendantes les unes par rapports aux autres, c'est à dire qu'elles sont distinctes et qu'elles ont un fonctionnement indépendant. Elles sont aussi mobiles les unes par rapport aux autres, de sorte qu'elles peuvent subir des déplacements au sein de la cavité 1 sans pour autant compromettre le fonctionnement du système endoscopique d'imagerie.

L'exemple de la figure 1 illustre un système d'imagerie comprenant 4 caméras (Cam0, Cam1, Cam2, Cam3). Chacune de ces caméras est insérée via le trocart 8, puis fixée dans la cavité 1 par tout moyen d'accroche connu, soit directement sur la paroi interne de la cavité 1, soit sur un organe proche du champ opératoire 2. Parmi les moyens d'accroche, les systèmes de ventouse seront particulièrement intéressants pour accrocher des caméras sur certains organes ou sur la paroi interne de la cavité 1. Chaque caméra pourra également être munie d'un aimant qui la plaque contre la paroi interne de la cavité 1, dès lors qu'un autre aimant est placé en regard de la caméra sur la paroi externe de la cavité. Outre la fonction d'accroche, cela permettra également de déplacer facilement la caméra si cela s'avère nécessaire pour une tâche opératoire particulière.

L'agencement des caméras est réalisé de manière à avoir un champ de vision cumulé couvrant l'ensemble du champ opératoire, de manière à pouvoir observer ce champ opératoire 2 selon plusieurs points de vue différents. L'agencement de ces caméras dépend bien entendu des dimensions du champ opératoire 2, mais également des caractéristiques techniques des caméras, tels que le champ de vision, la profondeur de champ et le type de focale utilisée.

On utilise au moins trois caméras de manière à pouvoir accéder à des zones cachées du champ opératoires (non visibles par un mono ou un stéréo endoscope) et augmenter les données concernant ce champ opératoire. Ceci permet d'augmenter le champ de vision virtuel du chirurgien en lui fournissant des informations supplémentaires sans pour autant bouger les caméras. Le système proposé n'est en outre pas soumis aux contraintes d'agencement caractérisant les systèmes de stéréo endoscopie, pour lesquels il est par exemple nécessaire que les deux caméras utilisées soient très proches l'une de l'autre. On pourra ainsi avoir des caméras relativement éloignées les unes des autres, ce qui n'est pas gênant pour reconstruire une représentation 3D du champ opératoire.

Deux parmi ces au moins trois caméras sont fixées sur l'un des outils (ou sur chaque outil) destiné à être inséré dans la cavité 1 par l'un des trocarts (5;6) prévus à cet effet. Ces caméras placées sur l'outil viennent en complément de la ou des caméra(s) placée(s) à l'intérieur de la cavité 1.

Dans le mode de réalisation illustré à la figure 1, les caméras sont introduites par un trocart 8 qui sera ensuite utilisé pour introduire un moyen de projection 9 d'une image texturée sur le champ opératoire. Ce moyen de projection 9 permet de renforcer la texture naturelle des organes observés par les caméras. Dans certains cas, ces organes sont en effet relativement homogènes, ce qui rend difficile l'identification de points d'intérêt (c'est en particulier le cas de la surface du foie). Or, de tels points sont importants dans notre approche. Comme on le verra plus loin, nous avons en effet besoin de matérialiser sur les organes des points susceptibles d'être vus par plusieurs caméras à la fois pour permettre une détermination de la position relative des caméras, et aussi pour permettre d'apparier la projection de ces points dans au moins deux caméras, ce qui permettra de reconstruire les coordonnées 3D de ces dits points.

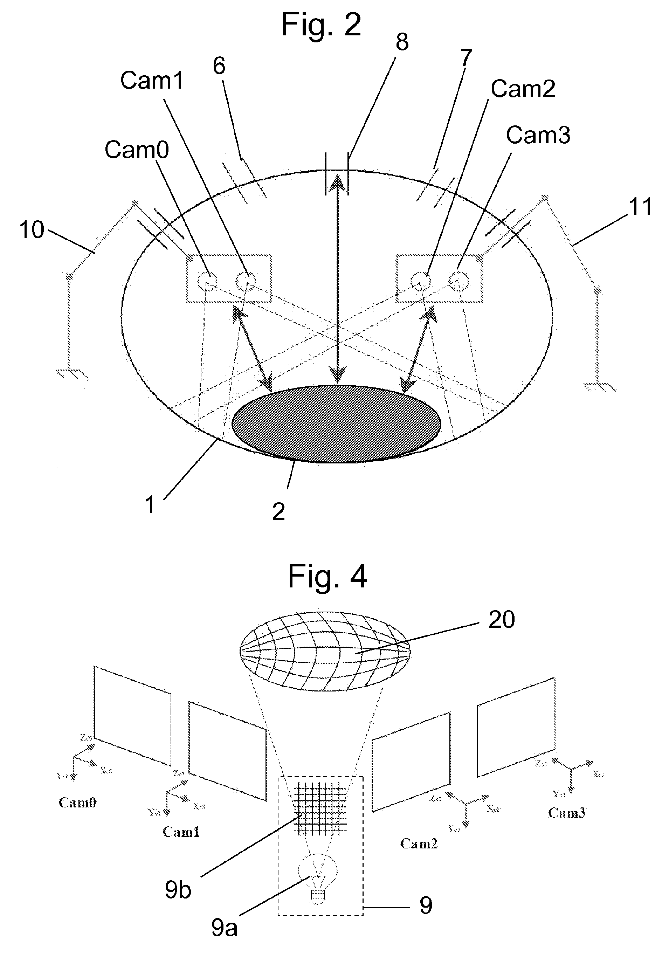

Comme cela est illustré à la figure 4, le moyen de projection 9 comporte une source lumineuse 9a (par exemple, une source de lumière froide classique en endoscopie, ou une diode électroluminescente). Cette source 9a éclaire la scène au travers d'un objet inhomogène 9b, qui crée donc une texture 20. Cet objet 9b peut par exemple être une lentille sur laquelle sont gravés des motifs. Les caractéristiques de la lentille et des motifs sont calculés pour obtenir lors de l'éclairage des organes par le système de projection 9 un motif aussi précis que possible, par exemple une grille. Il n'est cependant pas nécessaire que ce motif soit parfaitement net, il suffit qu'il apporte une information permettant d'apparier des points dans les diverses caméras qui observent la scène.

On notera qu'il est possible d'introduire les caméras et le moyen de projection 9 par l'un des orifices utilisés pour l'introduction des outils chirurgicaux, ce qui permet de supprimer un orifice cutané lorsqu'il n'est pas nécessaire pour une autre fin telle que la détermination relative des caméras. Il n'y a donc plus nécessairement un orifice dédié spécifiquement aux moyens de capture d'image 3 comme cela est le cas dans les systèmes endoscopiques d'imagerie connus.

Pour renforcer la texture naturelle des organes observés, il peut également être utilisé une source laser fibrée de couleur verte munie d'une optique diffractive spéciale. Cette dernière met en forme la lumière et permet de projeter un réseau de lignes ou de points sur la surface des organes du cham opératoire, de sorte que la lumière vient enrichir la texture naturelle des organes tout en dévoilant leurs formes. La couleur verte du laser est particulièrement appropriée dans le cadre de la chirurgie endoscopique. En effet, les images issues de la cavité abdominale par exemple ont tendance à révéler une couleur dominante dans les tons rouges ou rosés, la lumière verte apportant donc un contraste particulier. En outre, la solution du laser fibré permet de garder la source du laser en dehors de la cavité du champ opératoire. L'extrémité de la fibre optique est composée d'une petite armature comprenant l'optique diffractive spéciale. Cette armature est insérée dans la cavité abdominale à travers un trocart.

La fixation des caméras dans la cavité 1 peut également être réalisée par une structure mécanique dédiée. Un tel mode de réalisation est illustré par exemple à la figure 2. Dans ce mode de réalisation, on prévoit des incisions supplémentaires permettant de placer des trocarts à travers lesquels des structures mécaniques (10;11), sous la forme de bras articulés par exemple, sont insérées, et auxquelles sont fixées les caméras. Pour réduire les incisions, on pourra fixer plusieurs caméras orientées différemment sur le même bras.

Il peut également être prévu une armature déployable, adaptée pour pouvoir être insérée à travers un trocart puis déployée à l'intérieur de la cavité 1, formant ainsi une structure sur laquelle les caméras peuvent ensuite être fixées. Cette dernière solution présente l'avantage de ne pas avoir à poser un ou plusieurs trocart(s) dédiés à la structure.

L'insertion et le positionnement des caméras dans la cavité peuvent être effectués par un outil spécifique, composé d'un tube rigide comprenant éventuellement plusieurs canaux opérateurs. Les caméras sont stockées à l'intérieur de la partie distale du tube. Dans une des configurations possibles des caméras, il s'agit d'une tête stéréoscopique. Un mécanisme utilisant des câbles semi rigides permet de manipuler le support des caméras. Cet outil est compatible avec la plupart des trocarts utilisés en endoscopie classique. L'étanchéité est assurée par un bouchon ne laissant passer que les câbles d'alimentation et de contrôle de la tête stéréoscopique. Le déploiement et l'extraction sont donc assurés par un mécanisme simple à base de câbles semi rigides et assurant le pivotement de la tête stéréoscopique.

Les caméras placées dans la cavité 1 permettent d'observer en 3D une zone particulière du champ opératoire 2, selon le point de vue sélectionné, en fonction du couple de caméras choisi pour former le capteur stéréoscopique. L'utilisation d'une pluralité de caméras permet d'augmenter les points de vue virtuels offerts au praticien, sans qu'il n'ait besoin de modifier la position desdites caméras, ce qui est particulièrement avantageux puisque cela réduit les manipulations pendant les interventions.

L'image affichée par les moyens d'affichage 5, étant construite à partir d'informations obtenues depuis une multiplicité de points de vue, sera d'une qualité accrue par rapport aux systèmes existants qui offrent un point de vue unique (ou au mieux en conditions stéréoscopiques) du champ opératoire.

En outre, l'image est parfaitement stable puisqu'on dispose d'un modèle 3D permettant de traiter les informations des caméras pour en tirer des informations 3D permettant, par fusion des données de toutes les caméras, de synthétiser une image 2D ou 3D, selon le point de vue sous lequel le praticien veut l'observer.

Notons que la qualité de la reconstruction 3D dépend de la densité de points mis en correspondance, la qualité de l'image reconstruite étant d'autant meilleure que la densité de points utilisés est importante. Cette densité dépend des caractéristiques de la texture projetée sur le champ opératoire, ainsi que de la puissance de calcul disponible. Toutefois, puisque le praticien est intéressé essentiellement par une zone particulière du champ opératoire, on peut se contenter d'opérer une reconstruction 3D sur un nombre relativement restreint de points (par exemple, une grille de 100 lignes et de 100 colonnes), et réaliser la fusion des données des caméras uniquement sur la zone d'intérêt, en mettant en oeuvre des méthodes d'interpolation qui permettront de synthétiser une image d'une résolution satisfaisante selon le ou les points de vue souhaités. Cela permet en conséquence de gagner en temps de traitement de l'information sans perdre en qualité d'image puisque la zone d'intérêt est correctement reconstruite.

Un tel système d'imagerie présente en outre l'avantage d'éviter au praticien d'avoir recours à une commande mécanique des moyens de capture d'image pour observer telle ou telle zone du champ opératoire. En effet, le praticien a uniquement besoin d'indiquer le point de vue d'observation désiré (par tout type de commande, telle qu'une commande vocale, tactile, ou gyroscopique par exemple), sans avoir à modifier la position des caméras, et l'image correspondante est immédiatement synthétisée et affichée à l'écran. Cela permet donc également d'éviter un aide opératoire spécifiquement assigné à la visualisation du champ opératoire puisque le praticien a un contrôle complet et simple de la visualisation du champ opératoire.

Enfin, le système endoscopique d'imagerie proposé permet un gain de place important dans la salle d'opération, puisqu'il n'est plus nécessaire d'avoir un outil spécifique, tel qu'un robot porte endoscope, pour le contrôle du déplacement des moyens de capture d'image dans la cavité 1 en fonction de la zone d'observation désirée du champ opératoire.

Comme on l'a dit plus haut, chaque couple de caméras peut former un capteur stéréoscopique permettant d'obtenir un certains nombre d'informations 3D sur une zone du champ opératoire, de manière à construire une représentation 3D de cette zone particulière.

La reconstruction d'une image 3D d'une zone particulière du champ opératoire est particulièrement avantageuse puisqu'elle ne sollicite pas le cerveau du chirurgien. En effet, le chirurgien dispose directement d'une représentation virtuelle tridimensionnelle de la zone en question. Dans le cas de la cavité abdominale par exemple, l'information 3D obtenue grâce aux caméras permet de représenter en 3D la surface de la cavité abdominale. La retranscription visuelle sur moniteur passe par un rendu de la représentation 3D selon un point de vue virtuel contrôlable directement par le chirurgien. Pour donner un maximum de réalisme à la visualisation, la représentation 3D est texturée avec les images originales issues des caméras.

Pour faire de la reconstruction 3D, il est important de connaître la position relative des deux caméras formant le capteur stéréoscopique de manière à pouvoir corréler plus facilement les données acquises par les deux caméras.

Il existe plusieurs possibilités pour déterminer ce positionnement relatif des caméras les unes par rapport aux autres. Les caméras peuvent par exemple être portées par un dispositif mécanique adapté pour passer à travers le trocart et permettant, après introduction dans la cavité abdominale, un déploiement des caméras dans une position relative prédéterminée. La connaissance de la position relative des caméras peut aussi se faire par des algorithmes de traitement d'images particuliers. On utilisera la plupart du temps des moyens de détermination de la position relative des caméras spécialement prévus.

On pourra par exemple utiliser un dispositif de positionnement magnétique, positionné à l'intérieur ou à l'extérieur de la cavité 1, et permettant de déterminer la position exacte de chaque caméras. Un tel dispositif comportera par exemple une source de champ magnétique variable, située à l'extérieur du corps humain, et deux boucles de courant fixées sur chaque caméra. Les variations du champ induisent le passage d'un courant dans les boucles de courant, ce courant étant mesuré et permettant de déterminer la position et l'orientation de la caméra.

Une autre solution consiste à utiliser un dispositif de localisation optique. Ce dispositif est formé de manière à fournir trois points de référence, fixes les uns par rapport aux autres, au moins pendant le temps de l'acquisition d'image. On pourra aussi prévoir un élément indéformable placé dans la cavité 1 de manière à être dans le champ de vision des au moins deux caméras formant le capteur stéréoscopique considéré, voire par toutes les caméras placées dans la cavité 1, cet élément indéformable servant de référentiel à partir duquel le positionnement relatif des caméras peut être établi. Par élément indéformable, on entend un élément qui, une fois en position dans la cavité, ne subit pas de déformations de sorte qu'il sert de référentiel. Ainsi, on pourra prévoir un élément qui peut être déformé lors de son introduction dans la cavité, et dont la forme sera fixe une fois son déploiement effectué au sein de la cavité.

La localisation optique peut également consister en une caméra de référence, agencée de manière à pouvoir observer les caméras placées dans la cavité. A cette fin, on pourra utiliser une caméra placée à l'intérieur de la cavité ayant un champ de visualisation élargi de manière à pouvoir observer toutes les caméras. Cette caméra de référence est fixe ou à tout le moins a un positionnement qui peut être déterminé avec certitude par rapport à un référentiel fixe. On utilisera préférentiellement deux caméras de référence pour avoir une information plus précise. On pourra par exemple prévoir deux caméras de référence à l'intérieur de la cavité, chaque caméra de référence pouvant observer l'autre caméra de référence en plus des caméras dont le positionnement relatif est recherché. Une autre solution consiste à solidariser chacune des caméras placées à l'intérieur de la cavité à un organe indéformable de référence qui s'étend en dehors de la cavité, de manière à pouvoir être observé par la ou les caméra(s) de référence placé(s) dans ce cas en dehors de la cavité.

Il est aussi possible de déterminer la position relative des caméras en utilisant un moyen de projection d'une texture sur une paroi au niveau du champ opératoire, cette texture étant adaptée pour former un référentiel de base aux caméras. On pourra par exemple utiliser un projecteur 9 inséré via un trocart 8 dédié comme cela est représenté à la figure 1, qui projettera le motif considéré. Cette solution est en outre illustrée schématiquement à la figure 4. Comme précédemment, le motif projeté par ce moyen de projection 9 doit permettre de matérialiser au moins trois points sur le champ opératoire.

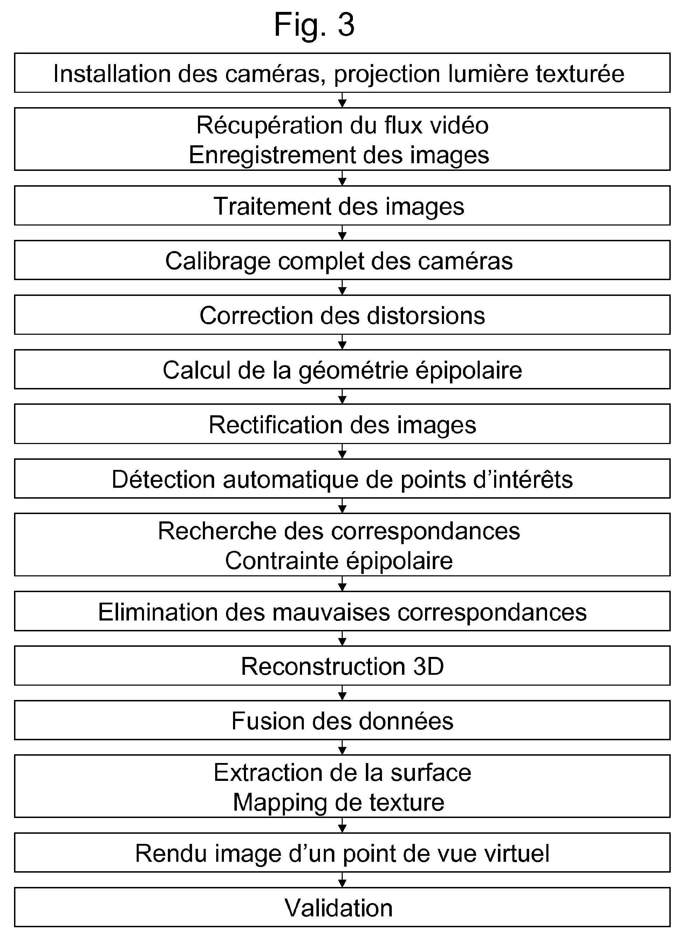

La figure 3 illustre le diagramme d'un mode de fonctionnement du système endoscopique d'imagerie, ou à tout le moins le diagramme de validation du système endoscopique d'imagerie lors de sa mise en place pour une utilisation au cours d'une intervention chirurgicale.

Ce diagramme se décompose selon les étapes suivantes, dont l'ordre n'a pas d'importance :
- Installation des caméras et de la lumière texturée
- Récupération du flux vidéo et enregistrement des images
- Traitement des images
- Calibrage complet des caméras
- Correction des distorsions
- Géométrie épipolaire
- Rectification éventuelle des images
- Détection de points d'intérêts (avec et sans lumière texturée)
- Recherche des correspondances de points avec contrainte épipolaire
- Elimination des mauvaises correspondances
- Reconstruction 3D par triangulation
- Fusion des données
- Extraction de la surface et mapping de texture
- Rendu image d'un point de vue virtuel
- Validation complète

Chacune des différentes étapes présentées ci-dessus peut être implémentée par différentes méthodes à la disposition de l'homme du métier. Pour ce faire, on pourra par exemple se référer aux documents suivants :
- La publication de E. Prados et O. Faugeras intitulée « Handbook of Mathematical Models in Computer Vision » (Springer, 2006) ;
- La publication de R. Horaud et F. Dornaika intitulée « Hand-Eye Calibration » (The International Journal of Robotics Research, Vol.14, N°3, pp.195-210, Juin 1995) ;
- La publication de R.I. Hartley et A. Zisserman intitulée « Multiple View Geometry in Computer Vision » (Cambridge University Press, 2000);
- La publication de O. Faugeras intitulée « Three-Dimensional Computer Vision - A Geometric Viewpoint » (The MIT Press, Cambridge, MA, USA, 1993) ;
- La publication de R. Horaud et O. Monga intitulée « Vision par ordinateur : outils fondamentaux » (Deuxième édition revue et augmentée, Editions Hermès, Paris, 1995) ;
- La publication de Z. Zhang intitulée « A flexible new technique for camera calibration » (IEEE Transactions on Pattern Analysis and Machine Intelligence, 22(11):1330-1334, 2000) ;
- La bibliothèque de traitement d'images « OPEN CV » (voir le site internet http://www.intel.com/technology/computing/opencv/)
- La bibliothèque de traitement d'images « Camera Calibration Toolbox for Matlab » (voir le site internet http://www.vision.caltech.edu/bouguetj/calib_doc/)

L'homme du métier adaptera aisément les méthodes décrites dans ces publications aux besoins de l'invention.

## Revendications

1. Système endoscopique d'imagerie pour l'observation d'un champ opératoire (2) dans un volume (1) situé à l'intérieur du corps d'un animal, comprenant :
- Des moyens de capture d'images (3) destinés à fournir des informations sur le champ opératoire (2), les moyens de capture (3) comprenant au moins deux dispositifs de capture d'images indépendants et mobiles l'un par rapport à l'autre, les dispositifs de capture d'images étant des caméras destinées à être positionnées dans le volume (1),
- Des moyens de traitement (4) des informations issus des moyens de capture, les moyens de traitement (4) comprenant des moyens de calcul pour l'obtention d'informations tridimensionnelles sur le champ opératoire (2) observé à partir des informations reçues des dispositifs de capture d'images, et des moyens de reconstruction d'une image tridimensionnelle d'une région d'intérêt du champ opératoire à partir des informations tridimensionnelles obtenues, et
- Des moyens d'affichage (5) des informations traitées par les moyens de traitement (4),
- au moins un outil destiné à être inséré dans le volume situé à l'intérieur du corps d'un animal,
**caractérisé en ce que** les moyens de capture d'images (3) comprennent au moins trois caméras, deux parmi ces trois caméras étant fixées sur l'outil.

2. Système selon la revendication 1, **caractérisé en ce que** les moyens de traitement (4) comprennent en outre des moyens de synthèse d'une image de la région d'intérêt, lesdits moyens de synthèse comprenant des moyens pour fusionner l'image tridimensionnelle reconstruite de la région d'intérêt avec des informations issues des caméras sur la région d'intérêt, lesdites informations étant interpolées au niveau de résolution souhaité.

3. Système selon l'une quelconque des revendications 1 ou 2, **caractérisé en ce que** la région d'intérêt correspond au champ opératoire dans son ensemble.

4. Système selon l'une quelconque des revendications 1 à 3, **caractérisé en ce qu'**il comprend en outre des moyens de sélection d'un point de vue, les moyens d'affichage (5) comprenant des moyens pour afficher l'image tridimensionnelle correspondant au point de vue sélectionné.

5. Système selon l'une quelconque des revendications 1 à4, **caractérisé en ce qu'**il comprend en outre des moyens de détermination de la position relative des caméras (cam0;cam1;cam2;cam3).

6. Système selon la revendication 5, **caractérisé en ce que** les moyens de détermination de la position relative comprennent un dispositif de positionnement magnétique pour localiser les caméras (cam0;cam1;cam2;cam3).

7. Système selon la revendication 5, **caractérisé en ce que** les moyens de détermination de la position relative comprennent un dispositif de localisation optique des caméras basé sur les informations issues des caméras (cam0;cam1;cam2;cam3).

8. Système selon la revendication 7, **caractérisé en ce que** le dispositif de localisation optique des caméras comprend un élément de référence agencé de manière à pouvoir être observé par au moins deux caméras (cam0;cam1;cam2;cam3).

9. Système selon la revendication 7, **caractérisé en ce que** le dispositif de localisation optique des caméras comprend au moins deux caméras de référence agencées de manière à pouvoir observer les autres caméras (cam0;cam1;cam2;cam3).

10. Système selon la revendication 9, **caractérisé en ce que** les caméras de référence sont situées à l'extérieur du volume (1), chacune des caméras (cam0;cam1;cam2;cam3) situées à l'intérieur du volume étant couplée à un organe indéformable, l'organe indéformable ayant une partie s'étendant à l'extérieur du volume de manière à pouvoir être observée par les caméras de référence.

11. Système selon la revendication 9, **caractérisé en ce que** les moyens de détermination de la position relative comprennent un moyen de projection d'une texture (9) dans le champ opératoire de manière à former un référentiel pour les caméras (cam0;cam1;cam2;cam3).

12. Système selon l'une quelconque des revendications 1 à 11, **caractérisée en ce que** les caméras (cam0;cam1;cam2;cam3) sont des caméras d'un volume inférieur à 5 mm³.

13. Système selon l'une quelconque des revendications 1 à 12, **caractérisée en ce que** les cameras qui ne sont pas fixées sur l'outil comprennent un dispositif d'accroche adapté pour une accroche de la caméra dans le volume (1).

14. Système selon la revendication 13, **caractérisé en ce que** le dispositif d'accroche comprend un premier élément fixé sur la caméra et un deuxième élément distinct, les premier et deuxième éléments pouvant être couplés par aimantation, le deuxième élément étant destiné à rester à l'extérieur du volume (1).

15. Système selon l'une quelconque des revendications 1 à 14, **caractérisée en ce qu'**il comprend en outre une structure mécanique destinée à être placée dans le volume (1) pour une accroche des cameras qui ne sont pas fixées sur l'outil.

16. Système selon la revendication 15, **caractérisé en ce que** la structure mécanique est une structure adaptée pour être insérée par voie endoscopique dans le volume (1) et pouvant être déployée dans le volume (1).

## Patentansprüche

1. Endoskopisches Bilddarstellungssystem für die Beobachtung eines Operationsfelds (2) in einem Volumen (1), das sich im Innern des Körpers eines Tiers befindet, umfassend:
- Bilderfassungsmittel (3), die bestimmt sind, Informationen über das Operationsfeld (2) zu liefern, wobei die Erfassungsmittel (3) wenigstens zwei voneinander unabhängige und bewegbare Bilderfassungsvorrichtungen umfassen, wobei die Bilderfassungsvorrichtungen Kameras sind, die bestimmt sind, in dem Volumen (1) positioniert zu sein,
- Verarbeitungsmittel (4) der von den Erfassungsmitteln bereitgestellten Informationen, wobei die Verarbeitungsmittel (4) Rechenmittel für den Erhalt von dreidimensionalen Informationen über das Operationsfeld (2) umfassen, das auf der Basis von Informationen, die von den Bilderfassungsvorrichtungen erhalten wurden, beobachtet wird, und Rekonstruktionsmittel eines dreidimensionalen Bilds einer interessierenden Region des Operationsfelds auf der Basis der erhaltenen dreidimensionalen Informationen, und
- Anzeigemittel (5) der von den Verarbeitungsmitteln (4) verarbeiteten Informationen,
- wenigstens ein Werkzeug, das bestimmt ist, in das Volumen eingeführt zu sein, das sich im Innern des Körpers eines Tiers befindet,
**dadurch gekennzeichnet, dass** die Bilderfassungsmittel (3) wenigstens drei Kameras umfassen, wobei zwei von diesen drei Kameras auf dem Werkzeug befestigt sind.

2. System nach Anspruch 1, **dadurch gekennzeichnet, dass** die Verarbeitungsmittel (4) ferner Synthesemittel eines Bilds der interessierenden Region umfassen, wobei die Synthesemittel Mittel umfassen, um das rekonstruierte dreidimensionale Bild der interessierenden Region mit von Kameras über die interessierende Region ausgegebenen Informationen zu verschmelzen, wobei die Informationen auf Ebene der gewünschten Auflösung interpoliert sind.

3. System nach einem der Ansprüche 1 oder 2, **dadurch gekennzeichnet, dass** die interessierende Region dem Operationsfeld insgesamt entspricht.

4. System nach einem der Ansprüche 1 bis 3, **dadurch gekennzeichnet, dass** es ferner Auswahlmittel eines Blickpunkts umfasst, wobei die Anzeigemittel (5) Mittel umfassen, um das dreidimensionale Bild anzuzeigen, das dem ausgewählten Blickpunkt entspricht.

5. System nach einem der Ansprüche 1 bis 4, **dadurch gekennzeichnet, dass** es ferner Bestimmungsmittel der relativen Position der Kameras (cam0; cam1; cam2; cam3) umfasst.

6. System nach Anspruch 5, **dadurch gekennzeichnet, dass** die Bestimmungsmittel der relativen Position eine Magnetpositionierungsvorrichtung umfassen, um die Kameras (cam0; cam1; cam2; cam3) zu lokalisieren.

7. System nach Anspruch 5, **dadurch gekennzeichnet, dass** die Bestimmungsmittel der relativen Position eine optische Lokalisierungsvorrichtung der Kameras umfassen, die auf den von den Kameras (cam0; cam1; cam2; cam3) ausgegebenen Informationen basiert.

8. System nach Anspruch 7, **dadurch gekennzeichnet, dass** die optische Lokalisierungsvorrichtung der Kameras ein Referenzelement umfasst, das derart angeordnet ist, dass es von wenigstens zwei Kameras (cam0; cam1; cam2; cam3) observierbar ist.

9. System nach Anspruch 7, **dadurch gekennzeichnet, dass** die optische Lokalisierungsvorrichtung der Kameras wenigstens zwei Referenzkameras umfasst, die derart angeordnet sind, dass sie die anderen Kameras (cam0; cam1; cam2; cam3) beobachten können.

10. System nach Anspruch 9, **dadurch gekennzeichnet, dass** sich die Referenzkameras außerhalb des Volumens (1) befinden, wobei jede der Kameras (cam0; cam1; cam2; cam3), die sich innerhalb des Volumens befinden, an ein unverformbares Organ gekoppelt ist, wobei das unverformbare Organ einen Teil hat, der sich außerhalb des Volumens derart erstreckt, dass der von den Referenzkameras beobachtbar ist.

11. System nach Anspruch 9, **dadurch gekennzeichnet, dass** die Bestimmungsmittel der relativen Position ein Projektionsmittel einer Textur (9) im Operationsfeld umfassen, um für die Kameras (cam0; cam1; cam2; cam3) einen Bezug herzustellen.

12. System nach einem der Ansprüche 1 bis 11, **dadurch gekennzeichnet, dass** die Kameras (cam0; cam1; cam2; cam3) Kameras eines Volumens unter 5 mm³ sind.

13. System nach einem der Ansprüche 1 bis 12, **dadurch gekennzeichnet, dass** die Kameras, die nicht an dem Werkzeug befestigt sind, eine Befestigungsvorrichtung umfassen, die für eine Befestigung der Kamera in dem Volumen (1) geeignet ist.

14. System nach Anspruch 13, **dadurch gekennzeichnet, dass** die Befestigungsvorrichtung ein erstes auf der Kamera befestigtes Element und ein zweites unterschiedliche Element umfasst, wobei das erste und zweite Element durch Magnetisierung koppelbar ist, wobei das zweite Element bestimmt ist, außerhalb des Volumens (1) zu bleiben.

15. System nach einem der Ansprüche 1 bis 14, **dadurch gekennzeichnet, dass** es ferner eine mechanische Struktur umfasst, die bestimmt ist, in dem Volumen (1) für eine Befestigung der Kameras platziert zu sein, die nicht auf dem Werkzeug befestigt sind.

16. System nach Anspruch 15, **dadurch gekennzeichnet, dass** die mechanische Struktur eine Struktur ist, die geeignet ist, um auf endoskopischem Weg in das Volumen (1) eingeführt zu sein und die in dem Volumen (1) entfaltbar ist.

## Claims

1. An endoscopic imaging system for observation of an operative site (2) within a volume (1) located inside the body of an animal, comprising:
- Image capture means (3) intended to provide information on the operative site (2), the image capture means (3) comprising at least two image capture devices that are independent and mobile relative to one another, the image capture devices being cameras intended to be positioned inside the volume (1),
- Processing means (4) to process data provided by the image capture means, the processing means (4) comprising computing means to obtain three-dimensional data on the observed operative site (2) based on data received from the image capture devices, and reconstruction means to reconstruct a three-dimensional image of a region of interest in the operative site based on the obtained three-dimensional data, and
- Display means (5) to display data processed by the processing means (4),
- At least an instrument intended to be inserted inside the volume located inside the body of an animal,
**characterized in that** the image capture means (3) comprise at least three cameras, two of these three cameras being fixed to the instrument.

2. The system of claim 1, **characterized in that** the processing means (4) further comprise synthesis means to synthesize an image of the region of interest, said synthesis means comprising means to merge the reconstructed three-dimensional image of the region of interest with data derived from the cameras on the region of interest, said data being interpolated to the desired resolution.

3. The system of any one of claim 1 or 2, **characterized in that** the region of interest corresponds to the whole operative site.

4. The system of any one of claim 1 to 3, **characterized in that** it further comprises means to select a viewing angle, the display means (5) comprising means to display the three-dimensional image corresponding to the selected viewing angle.

5. The system of any one of claim 1 to 4, **characterized in that** it further comprises means to determine the relative position of the cameras (cam0;cam1;cam2;cam3).

6. The system of claim 5, **characterized in that** the means to determine the relative position comprise a magnetic positioning device to locate the cameras (cam0;cam1;cam2;cam3).

7. The system of claim 5, **characterized in that** the means to determine the relative position comprise an optic locating device to locate the cameras, based on data derived from the cameras (cam0;cam1;cam2;cam3).

8. The system of claim 7, **characterized in that** the optic locating device for the cameras comprises a reference element arranged so that it can be observed by at least two cameras (cam0;cam1;cam2;cam3).

9. The system of claim 7, **characterized in that** the optic locating device for the cameras comprises at least two reference cameras arranged so that they are able to observe the other cameras (cam0;cam1;cam2;cam3).

10. The system of claim 9, **characterized in that** the reference cameras are positioned outside the volume (1), each of the cameras (cam0;cam1;cam2;cam3) positioned inside the volume being coupled with a non-deformable member, the non-deformable member having one part extending outside the volume so that it can be observed by the reference cameras.

11. The system of claim 9, **characterized in that** the means to determine the relative position comprise means (9) to project a texture onto the operative site, so as to form a reference system for the cameras (cam0;cam1;cam2;cam3).

12. The system of any one of claim 1 to 11, **characterized in that** the cameras (cam0;cam1;cam2;cam3) are cameras having a volume of less than 5 mm³.

13. The system of any one of claim 1 to 12, **characterized in that** the cameras that are not fixed to the instrument comprise an attachment device adapted to attach the camera inside the volume (1).

14. The system of claim 13, **characterized in that** the attachment device comprises a first element fixed to the camera and a second separate element, the first and second elements able to be magnetically coupled, the second element being intended to remain outside the volume (1).

15. The system of any one of claim 1 to 14, **characterized in that** it further comprises a mechanical structure intended to be placed inside the volume (1) for attachment of the cameras that are not fixed to the instrument.

16. The system of claim 15, **characterized in that** the mechanical structure is a structure adapted to be inserted via endoscopic route into the volume (1) and able to be deployed inside the volume (1).
